# EUROPÄISCHE PATENTSCHRIFT

(11) **EP 2 679 174 B1**
(45) Veröffentlichungstag und Bekanntmachung des Hinweises auf die Patenterteilung: **19.11.2014**
(21) Anmeldenummer: 13163333.1
(22) Anmeldetag: 11.04.2013
(51) Int. Cl.: A61B 17/16, A61B 17/32, A61B 17/3207

(54) **Medizinisches Instrument zum Abtrennen von Gewebe und Knorpel**
Medical instrument for severing tissue and cartilage
Instrument médical pour la séparation de tissus et de cartilages

(30) Priorität: 12.04.2012 DE 102012103153
(43) Veröffentlichungstag der Anmeldung: 01.01.2014
(73) Patentinhaber: Karl Storz GmbH & Co. KG, 78532 Tuttlingen (DE)
(72) Erfinder: Steinwachs, Matthias, Dr., 8704 Herrliberg (CH); Berberich, Sascha, 78532 Tuttlingen (DE)
(74) Vertreter: Witte, Weller & Partner Patentanwälte mbB

(56) Entgegenhaltungen:
- EP-A1- 1 882 455
- US-A- 4 811 734
- US-A1- 2003 163 126
- US-A1- 2011 270 294

## Beschreibung

Die Erfindung betrifft ein medizinisches Instrument zum Abtrennen von Gewebe und Knorpel, mit einem Außenschaft, der im Bereich seines distalen Endes zumindest ein Fenster mit zumindest einer Schneide aufweist, mit einem im Außenschaft aufgenommenen um seine Längsachse rotierbaren Innenschaft, der an seinem distalen Ende, im Bereich des Fensters des Außenschafts, zumindest eine Öffnung mit einer Schneide aufweist, die mit der zumindest einen Schneide des Außenschaftes schneidend zusammenwirkt, wobei der hohle Innenschaft an eine Unterdruckquelle anschließbar ist.

Ein derartiges Instrument ist bspw. aus der EP 1 882 455 A1 bekannt.

Derartige Instrumente werden in der minimal-invasiven Chirurgie zum Abtrennen von Gewebe im menschlichen oder tierischen Körper verwendet. Dazu wird das distale Ende des Außenschafts zu dem Operationsgebiet geführt, in dem sich das abzutrennende Gewebe befindet. Zum Abtrennen des Gewebes wird der Innenschaft mittels eines externen oder internen Antriebes in Rotation versetzt. Die am Innenschaft ausgebildeten Schneiden wirken beim Umlaufen mit einem als Schneide ausgebildeten Rand des Fensters des Außenschaftes schneidend zusammen, in dem die Schneide einer Öffnung des Innenschafts an der Schneide des Fensters im Außenschaft bei jedem Umlauf vorbeiläuft. Um das abzutrennende Gewebe zwischen die zusammenwirkenden Schneiden zu bringen, ist der Innenschaft mit einer Unterdruckquelle verbindbar, deren Saugwirkung durch den Innenschaft bis hin zu dem Fenster am Außenschaft reicht, um das abzutrennende Gewebe durch das Fenster in den Schaft zu saugen, so dass die Schneiden das Gewebe abtrennen können. Durch den Unterdruck wird das abgetrennte Gewebe durch den hohlen Innenschaft abgesaugt und somit von der Operationsstelle weggeführt.

Das Dokument US 20030163126, das sich dem Oberbegriff des Anspruchs 1 entspricht, offenbart ein medizinisches Instrument zum Abtrennen von Gewebe und Knorpel mit einem Außenschaft, der im Bereich seines distalen Endes zumindest ein erstes Fenster mit zumindest einer Schneide aufweist, mit einem im Außenschaft aufgenommenen um seine Längsachse rotierbaren Innenschaft, der an seinem distalen Ende, im Bereich des ersten Fensters des Außenschafts zumindest eine Öffnung mit einer Schneide aufweist, die mit der zumindest einen Schneide des Außenschafts schneidend zusammenwirkt, wobei der hohle Innenschaft an eine Unterdruckquelle anschließbar ist, wobei am Außenschaft im Bereich des distalen Endes ein weiteres Fenster ausgespart ist.

Insbesondere in der Arthroskopie liegen unter dem Hautgewebe relativ feste Knorpelbereiche, die bei Knorpeldefekten bis auf den Knochen hin entfernt werden müssen.

Bei der sog. Autologen Chondrozyten-Transplantation (ACD) zur Regenerierung eines Knorpels, ist es erforderlich, die Defektränder äußerst scharf abzugrenzen. Dies ist deswegen notwendig, damit ein an die Defektstelle verbrachtes Implantat an diesen Defekträndern nachhaltig anwachsen kann.

Mit dem eingangs erwähnten Instrument können keine solchen scharfen Defektränder bewerkstelligt werden und es wurde auch festgestellt, dass Knorpel bis hin auf die Knochenfläche nicht vollständig entfernt werden können.

Daher wird dann ein weiteres Instrument herangezogen, nämlich eine sog. Kürette.

Eine Kürette ist ein schaftartiges Instrument, das an seinem distalen Ende seitlich vorstehend eine umlaufende Schneidekante aufweist. Die Kontur und die Größe der Schneidekante werden so gewählt, dass diese die Defektstelle umrunden kann. Die Kürette wird in das Knorpelgewebe bis auf Höhe des Knochens eingedrückt. Mittig ist in der Kürette eine Öffnung über die, mittels eines Spatels, die innerhalb der umlaufenden Schneidekante der Kürette abgetrennten Gewebe- und Knorpelteile abgeschabt und entfernt werden können. Dabei lässt sich nicht vermeiden, dass das sog. Debridement, also abgetrennte Knorpelstücke ins Gelenk freigesetzt werden, was anschließend zu einer Gelenkspülung zur Entfernung der kleinen Stücke, sog. Chips, führen muss. Der Durchmesser der Kürette ist bei einer relativ großen Defektstelle dann entsprechend groß.

Es wurden schon Versuche dahingehend gemacht, eine solche Kürette als Saugkürette auszubilden. Dann müssen aber die von der Kürette abgetrennten Knorpelstücke immer kleiner sein als der Saugquerschnitt des Instrumentes, da ansonsten der Saugquerschnitt durch die Knorpelstücke verstopft. Dementsprechend müsste dann der Absaugkanal einen entsprechend großen Durchmesser aufweisen, was bei Defektstellen mit Durchmessern von einigen Zentimetern nicht durchführbar ist, da ein solch großer Raum nicht bei Gelenkoperationen zur Verfügung steht.

Eine Kürette mit Absaugung unter Zuhilfenahme eines von distal nach proximal gerichteten Flüssigkeitsstrahles ist aus der WO 2004/037095 A2 bekannt.

Wie eingangs erwähnt, werden diese Eingriffe meist minimal-invasiv durchgeführt. Es müssen nacheinander zwei unterschiedliche Instrumente der Operationsstelle zugeführt werden, zunächst die eingangs erwähnte Vorrichtung, ein sog. Shaver-Blade und anschließend eine Kürette gefolgt von einer Gelenkspülung.

Es ist daher Aufgabe der vorliegenden Erfindung hier Abhilfe zu schaffen und ein medizinisches Instrument zum Abtrennen von Gewebe und Knorpel zu entwickeln, das einfacher und effektiver zum Abtrennen von Gewebe und Knorpel einsetzbar ist.

Erfindungsgemäß wird die Aufgabe dadurch gelöst, dass am Außenschaft, im Bereich des distalen Endes, ein weiteres Fenster ausgespart ist, das von einer Kürette umgeben ist.

Diese Maßnahme hat nun den Vorteil, dass das Instrument am distalen Ende auf einer Seite als Shaver-Blade ausgebildet ist, durch das die relativ weichen Gewebestücke abgetrennt und abgesaugt werden können, und zugleich als Kürette ausgebildet ist, die um eine Defektstelle legbar und in das Gewebe, also Bindegewebe und Knorpel, bis auf Höhe des Knochens eindrückbar ist. Die von der Kürette abgetrennten relativ großen Gewebe- und Knorpelstücke können durch das weitere Fenster im Außenschaft in Richtung Innenschaft gesaugt werden und werden dort von den rotierenden Schneidekanten der Öffnungen im Innenschaft zerkleinert. Anschließend können diese zerkleinerten Stücke in den Innenraum des Innenschafts eingesaugt und abgeführt werden. Daher kann die Kürette einen Bereich umgrenzen, der wesentlich größer ist als der Durchmesser des Innenschaftes ist. Dies führt deswegen zu keinen Verstopfungen, da der rotierende Innenschaft, die durch die Kürette abgesaugten Knorpelstücke im Bereich des weiteren Fensters zunächst zerkleinert und erst dann die zerkleinerten Stücke in den Innenschaft eingesaugt werden.

Beim operativen Eingriff wird das Instrument zunächst im Bereich des Shaver-Blade-Fensters des Außenschaftes an die Defektstelle angelegt und es werden die weicheren Bindegewebs- und Knorpelteile abgetrennt und abgesaugt. Danach braucht das Instrument nur soweit um dessen Längsachse verdreht werden, bis die Kürette über der Defektstelle zum Liegen kommt. Diese wird dann in das Knorpelgewebe eingedrückt und trennt die noch verbleibenden Knorpelstücke im Umfangsbereich innerhalb der Kürette ab. Diese werden ebenfalls angesaugt, zerkleinert und dann abgeführt. Es ist auch möglich, das Instrument nur als Shaver-Blade oder nur als Kürette einzusetzen. Da die Kürette über der Defektstelle liegt und die Defektstelle zumindest größtenteils umrundet, und die abgetrennten Knorpelstücke in Richtung des rotierenden Innenschafts abgesaugt werden, besteht nicht die Gefahr, dass Knorpelstücke im Gelenk freigesetzt werden und dann zwangsläufig eine Gelenkspülung durchgeführt werden muss.

Somit wird nicht nur der Einsatz von zwei verschiedenen Geräten vermieden, die nacheinander eingeschoben und von der Operationsstelle entfernt werden müssen, sondern es kann auch die Knochenspülung weggelassen werden.

Bei sehr kleinen Knorpelteilen, bspw. bei einer Defektstelle im Knie, besteht immer die Gefahr, dass die Knorpelteile auch bei einem Spülvorgang nicht vollständig abgespült, sondern irgendwo im Kniegelenk zwischen die beiden aneinanderliegenden Knochen von Ober- und Unterschenkel hängenbleiben. Somit ist das Instrument einfacher zu handhaben und auch die damit durchgeführten Vorgänge sind für den Patienten wesentlich atraumatischer und im Ergebnis sicherer durchführbar.

In einer weiteren Ausgestaltung der Erfindung steht die Kürette von einer Außenfläche des Außenschafts radial vor.

Diese Maßnahme hat den Vorteil, dass diese Ausgestaltung dem einer üblichen Kürette entspricht, so dass der Operateur das Instrument, wenn dessen Kürette zum Einsatz kommt, so handhaben kann, wie er das beim Handhaben von Küretten als Einzelinstrument gewohnt ist. Er kann das Instrument, meist sogar unter Sichtkontrolle, bis an die Defektstelle vorschieben und, wenn er dann die Kürette zur Operationsstelle hindreht, diese so anlegen, dass sie die Defektstelle entsprechend umrundet und dann durch ein seitliches Bewegen diese in den Knorpel bis auf den Knochen eindrücken kann.

In einer weiteren Ausgestaltung der Erfindung ist die Kontur der Kürette, radial von außen auf den Außenschaft gesehen, U-förmig.

Diese Maßnahme hat den Vorteil, dass beim Ansetzen der Kürette an die Defektstelle über die offene Seite des U in diesen inneren Bereich noch eingesehen werden kann. Das erleichtert erheblich die Handhabung, insbesondere das Ansetzen an der Defektstelle. Dabei ist zu berücksichtigen, dass Knochenoberflächen, insbesondere bei den häufig in Schulter- oder Kniegelenk vorkommenden Defektstellen, stark gekrümmt sind. Eine solche U- oder hufeisenförmige Kontur erlaubt ein zielgerechtes Ansetzen an einer solchen Defektstelle.

In einer weiteren Ausgestaltung der Erfindung liegt das offene Ende der U-Kontur proximalseitig.

Diese Maßnahme hat den Vorteil, dass der Operateur beim Heranführen des Instrumentes an die Defektstelle von proximal nach distal blickend in den inneren Bereich der Kürette über das proximalseitig offene Ende Einblick hat, somit diese zielgerecht ansetzen kann.

Wie zuvor erwähnt, weisen die meisten Küretten eine kreisförmige oder ovale geschlossene umlaufende Trennkante auf. Je nach Ausgestaltung und Einsatzgebiet, kann die Trennkante in einer Ebene liegen oder entsprechend gekrümmt sein.

In Zusammenhang mit der U-Struktur ist von Vorteil, wenn die Trennkante der Kürette in einer Ebene liegt. Da der Operateur Einsicht über das offene Ende des U in die Defektstelle hat, kann er dann die Trennkante der Kürette durch entsprechende seitliche Kippbewegungen um die Längsachse des Schaftes entsprechend auch bei gekrümmten Oberflächen effektiv eindrücken. Effektiv bedeutet, dass eine sehr gerade Schnittkante am Umfang des Defektes entsteht, die eine wesentliche Voraussetzung für ein gutes und rasches Anwachsen eines entsprechenden Implantates ist.

In einer weiteren Ausgestaltung der Erfindung steht die Trennkante der Kürette um eine solche Höhe radial vor, die zumindest der Dicke einer abzutrennenden Knorpelschicht entspricht.

Diese Maßnahme hat den Vorteil, dass der Operateur die Kürette seitlich kräftig in das manchmal sehr zähe Knorpelgewebe eindrücken kann, bis er auf die Knochenoberfläche trifft. Dadurch ist sichergestellt, dass sämtlicher Knorpel abgetrennt werden kann. Entspricht diese Höhe den üblichen Knorpelschichten kann auch ausgeschlossen werden, dass die Trennkante bei starkem Drücken versehentlich zu weit in den Knochen eingetrieben wird, was unerwünscht ist.

In einer weiteren Ausgestaltung der Erfindung weist der Außenschaft ein einziges Fenster auf, das diametral gegenüberliegend zur Kürette angeordnet ist.

Dies hat den Vorteil, dass bei relativ dünnen Schäften das einzige Fenster des Shaver-Blades dann relativ groß ausgebildet werden kann, so dass die Stabilität des Schaftes in diesem distalen Endbereich durch dieses Fenster nicht so geschwächt wird, dass dann beim Eintreiben der Kürette Verformungen auftreten. Dadurch, dass das nur eine Fenster diametral gegenüberliegend zur Kürette ist, verbleiben zwischen diesem einen Fenster des Shaver-Blades und dem weiteren Fenster im Bereich der Kürette ausreichende Materialstege, um die Stabilität aufrechtzuerhalten. Außerdem gibt es dem Operateur ein gutes Gefühl, dass er nach dem ersten Operationsabschnitt mit dem Shaver-Blade den Außenschaft gerade um 180° drehen muss, um dann die Kürette einsatzbereit an die Defektstelle bringen zu können. Dies erleichtert die Handhabung.

In einer weiteren Ausgestaltung der Erfindung ist das Fenster des Shaver-Blades im Außenschaft durch einen von proximal nach distal radial nach innen geneigten Schrägschnitt des hohlen Außenschaftes ausgebildet.

Diese an sich bekannte Maßnahme hat den Vorteil, dass beim ausgänglichen Abtrennen des relativ weichen Bindegewebes, das distale Ende des Außenschaftes parallel zu diesem Schrägschnitt angelegt werden kann, so dass dann der innere rotierende Innenschaft effektiv dieses Bindegewebe abtrennen kann.

Dazu ist von Vorteil, dass der Innenschaft im Bereich des Schrägschnittes aus dem Fenster ragt.

Dies erlaubt besonders effizientes Abtrennen des Bindegewebes.

In einer weiteren Ausgestaltung der Erfindung weist die Umfangskante des Fensters des Außenschafts von dieser hochstehende Zacken auf.

Diese ebenfalls an sich bekannte Maßnahme hat den Vorteil, dass über diese Zacken des Shaver-Blades, das Instrument im Bereich des Fensters im Außenschaft unverrückbar in das Gewebe eingedrückt werden kann.

In einer weiteren Ausgestaltung der Erfindung weist der rotierbare Innenschaft mehrere Öffnungen auf.

Diese ebenfalls an sich bekannte Maßnahme hat den Vorteil, dass durch die mehreren Fenster, die ggf. auch unterschiedliche Größen und Geometrien aufweisen können, sowohl die abgetrennten Bindegewebs- als auch die durch die Kürette abgetrennten Knorpelstücke in kleinste Teile zertrennt werden können, die problemlos dann durch den Innenschaft abgesaugt werden können. Dadurch ist die Gefahr des Blockierens des Innenschaftes durch Gewebe- oder Knorpelstücke ausgeschlossen.

Als besonders vorteilhaft hat sich dabei erwiesen, wenn im Innenschaft drei solche Öffnungen vorhanden sind.

In einer weiteren Ausgestaltung der Erfindung weist der Außenschaft am proximalen Ende ein Kopplungsstück auf, durch das der Innenschaft von proximal führbar ist.

Das Kopplungsstück ermöglicht es, den Außenschaft an Griffteile, die ergonomisch von der Hand eines Operateurs ergriffen werden können, angekoppelt zu werden. Gleichzeitig dient dieses Kopplungsstück als Führung zum Einführen des Innenschaftes in den Außenschaft.

In einer weiteren Ausgestaltung der Erfindung weist der Innenschaft am proximalen Ende ein Anschlussstück auf, über das er mit einer Unterdruckquelle verbindbar ist.

Diese Maßnahme hat den Vorteil, dass zum eigentlichen operativen Eingriff dann eine Unterdruckquelle, bspw. über einen Schlauch, an den Innenschaft angeschlossen werden kann.

Nach einem operativen Eingriff kann dieser Schlauch entfernt werden, so dass auch der Innenraum des Innenschaftes entsprechend gereinigt werden kann.

In einer weiteren Ausgestaltung der Erfindung ist das Anschlussstück über Kupplungselemente mit einem Antrieb kuppelbar, der den Innenschaft um dessen Längsachse dreht.

Diese Maßnahme hat den Vorteil, dass über diese Kupplungselemente, z.B. radial vorspringende Kupplungszapfen, einfach eine Verbindung mit dem Antrieb geschaffen werden kann, der den Innenschaft dreht.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels im Zusammenhang mit den beiliegenden Zeichnungen näher beschrieben und erläutert. Es zeigen:
- Fig. 1: eine Seitenansicht eines erfindungsgemäßen medizinischen Instrumentes, wobei der Anschluss an eine Unterdruckquelle angedeutet ist,
- Fig. 2: eine der Darstellungen von Fig. 1 vergleichbare Seitenansicht in Explosionsdarstellung, d.h. der Innenschaft ist aus dem Außenschaft entnommen,
- Fig. 3: eine perspektivische Explosionsansicht von distal nach proximal von Innenschaft und davon abgenommenen Außenschaft, wobei das distale Ende des Außenschafts in zwei um 180° um die Längsachse verdrehte Positionen dargestellt ist, so dass einmal das erste Fenster im Außenschaft und zum anderen das weitere Fenster, das von der Kürette umgeben ist, ersichtlich sind,
- Fig. 4: eine stark vergrößerte Seitenansicht entsprechend der Darstellung von Fig. 1 des distalen Endes des Außenschaftes ohne Innenschaft,
- Fig. 5: eine vergleichbare Darstellung mit eingeschobenem Innenschaft,
- Fig. 6: eine ebenfalls stark vergrößerte perspektivische Ansicht des distalen Endes des Außenschaftes im Bereich der Kürette, ohne eingeschobenem Innenschaft,
- Fig. 7: eine der Fig. 6 vergleichbare Darstellung mit eingeschobenem Innenschaft,
- Fig. 8: stark schematisiert einen Oberschenkelknochen im Bereich des Kniegelenkes mit einer Defektstelle, nachdem diese durch den Shaver-Blade-Bereich des Instrumentes behandelt worden ist, und
- Fig. 9: nachdem die Defektstelle mit der Kürette bearbeitet worden ist.

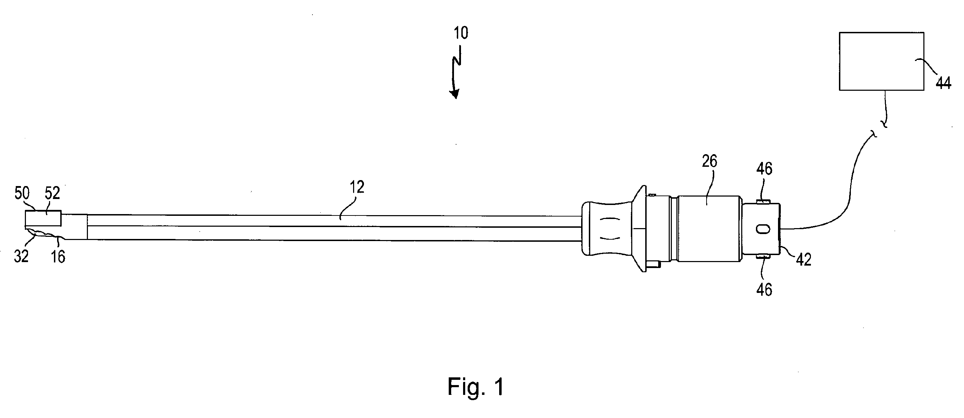

Ein den in den Figuren 1 bis 7 dargestelltes erfindungsgemäßes medizinisches Instrument zum Abtrennen von Gewebe und Knorpel ist in seiner Gesamtheit mit der Bezugsziffer 10 bezeichnet.

Das Instrument 10 weist einen lang erstreckten geradlinigen hohlen Außenschaft 12 auf.

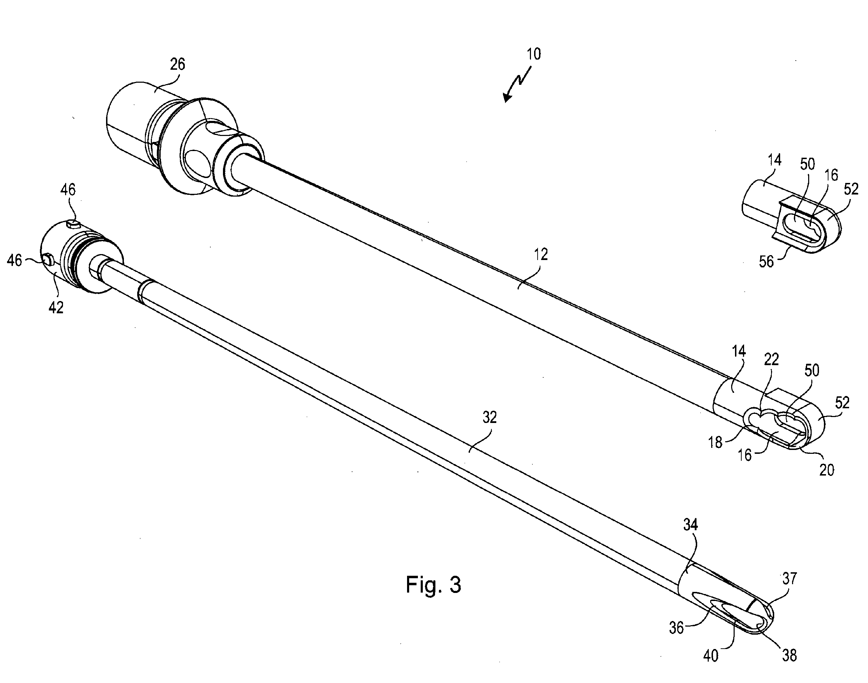

Dieser weist im Bereich seines distalen Endes 14, wie das insbesondere aus Fig. 3 ersichtlich ist, ein erstes Fenster 16 auf.

Das erste Fenster 16 weist eine Umfangskante 18 auf, die als Schneide 20 ausgebildet ist.

Von der Schneide 20 stehen Zacken 22 hoch, wie das insbesondere aus Fig. 3, 4 und 5 ersichtlich ist.

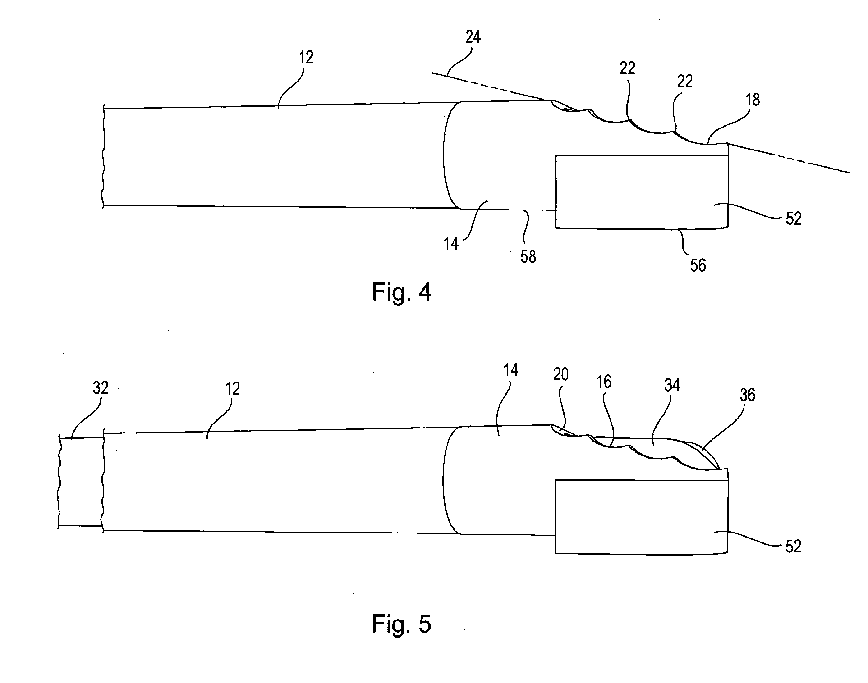

Die Umfangskante 18 liegt im Bereich eines Schrägschnittes 24, wie er in Fig. 4 angedeutet ist. Dieser Schrägschnitt 24 ist von proximal nach distal gesehen radial nach innen geneigt, so dass das erste Fenster 16 einen etwa ovalen Querschnitt aufweist. Die Neigung des Schrägschnittes 24 ist dabei, wie das aus Fig. 4 ersichtlich, dass am distalen Scheitelpunkt weniger als die Hälfte eines Durchmessers abgeschnitten ist.

Durch nachträgliches Bearbeiten oder schon bei Anfertigen des Schrägschnittes können die Zacken 22 herausgearbeitet werden.

Im Außenschaft 12 ist ein Innenschaft 32 aufgenommen.

Der Innenschaft 32 ist ebenfalls als lang erstreckter Hohlschaft ausgebildet, dessen Außendurchmesser in etwa dem lichten Innendurchmesser des Außenschaftes 12 entspricht.

Im Bereich seines distalen Endes 34 ist der Innenschaft 32 umfänglich etwa gleichmäßig verteilt mit drei Öffnungen 36, 37 und 38 versehen.

Jede der Öffnungen 36, 37 und 38 ist an seiner Umfangskante mit einer umlaufenden Schneide 40 versehen, wobei lediglich die Schneide 40 der Öffnung 36 mit einem Bezugszeichen versehen ist. Die Länge des Innenschaftes 32 ist so gewählt, dass er von proximal komplett in den Außenschaft 12 eingeschoben werden kann.

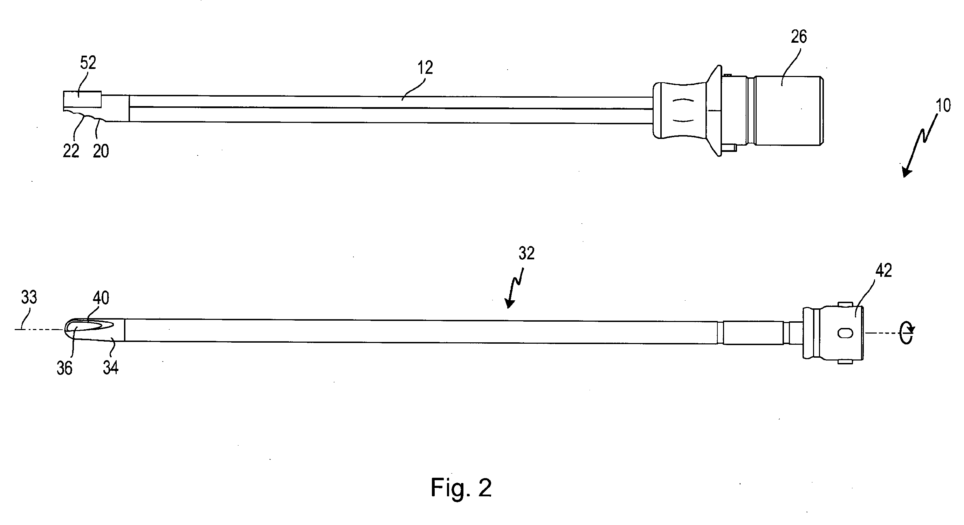

Dabei liegen dann die drei Öffnungen 36, 37 und 38 auf Höhe des ersten Fensters 16 im Außenschaft 12, wie das aus der Explosionsdarstellung von Fig. 2 ersichtlich ist. Aus Fig. 5 ist ersichtlich, dass das distale Ende 34 des Innenschaftes 32 mit seinen Öffnungen seitlich etwas aus dem ersten Fenster 16 herausragt.

Wird der Innenschaft 32 um seine Längsachse 33 gedreht, die auch zugleich die Längsachse des Instrumentes 10 und die des Außenschaftes 12 ist, drehen sich die Schneiden 40 der Öffnungen 36 etc. mit und laufen an der ebenfalls als Schneide 20 ausgebildeten Umfangskante 18 des ersten Fensters 16 im Außenschaft 12 vorbei. Dabei finden dann scherenartige Schnitte statt.

Am proximalen Ende weist der Innenschaft 32 ein Anschlussstück 42 auf, über das der Innenschaft 32 an eine Unterdruckquelle 44 angeschlossen werden kann, wie das in Fig. 1 angedeutet ist.

Darüber hinaus weist das Anschlussstück 42 noch radial vorstehende Kupplungszapfen 46 auf, über die ein Kupplungsstück eines hier nicht näher dargestellten Antriebes gebracht werden kann, durch den der Innenschaft 32 im Außenschaft 12 gedreht werden kann.

Der Außenschaft 12 weist an seinem proximalen Ende ein Kopplungsstück 26 auf. Der Innenschaft 32 kann von proximal nach distal durch das Kopplungsstück 26 so weit hindurchgeschoben werden, bis das Anschlussstück 42 passend im Inneren des hülsenartigen Körpers des Kopplungsstückes 26 eingetreten ist, wie das aus Fig. 1 ersichtlich ist. Dadurch ist der Innenschaft 32 im Außenschaft 12 geführt und aufgenommen.

Der distale Bereich des Instrumentes 10 arbeitet als ein sog. "Shaver-Blade".

Dadurch dass der Innenschaft 32 im Bereich des ersten Fensters 16 des Außenschaftes 12 teilweise herausragt, wie das in Fig. 5 dargestellt ist, kann ein besonders aggressives Schneiden von Gewebe durchgeführt werden.

Dazu wird die Umfangskante 18, die mit den Zacken 22 versehen ist, an das Gewebe angelegt. Beim Drehen des Innenschaftes 32 trennen die Schneiden 40 der Öffnungen 36, 37, 38 in Zusammenwirken mit der Schneide 20 des ersten Fensters 16 im Außenschaft entsprechende Gewebestücke ab und diese werden in den Innenraum des Innenschafts 32 durch die Öffnungen 36, 37, 38 eingesaugt und abgeführt. Der Unterdruck sorgt dafür, dass die abzutrennenden Gewebepartien seitlich an das erste Fenster 16 im Außenschaft 12 herangesaugt und dann abgetrennt werden.

Auf der dieser "Shaver-Blade"-Konstruktion diametral gegenüberliegenden Seite, ist am Außenschaft 12 ein weiteres etwa ovales Fenster 50 ausgespart, wie das insbesondere aus Fig. 3, 6 und 7 ersichtlich ist.

Dieses weitere Fenster 50 ist von einer Kürette 52 umgeben, die die Kontur 54 eines nach proximal offenen U' hat. Die radial äußere U-Kante ist als scharfe Trennkante 56 ausgebildet. Wie insbesondere aus Fig. 4 und 5 ersichtlich, steht diese Trennkante 56 seitlich über die Außenfläche 58 des Außenschaftes 12 vor.

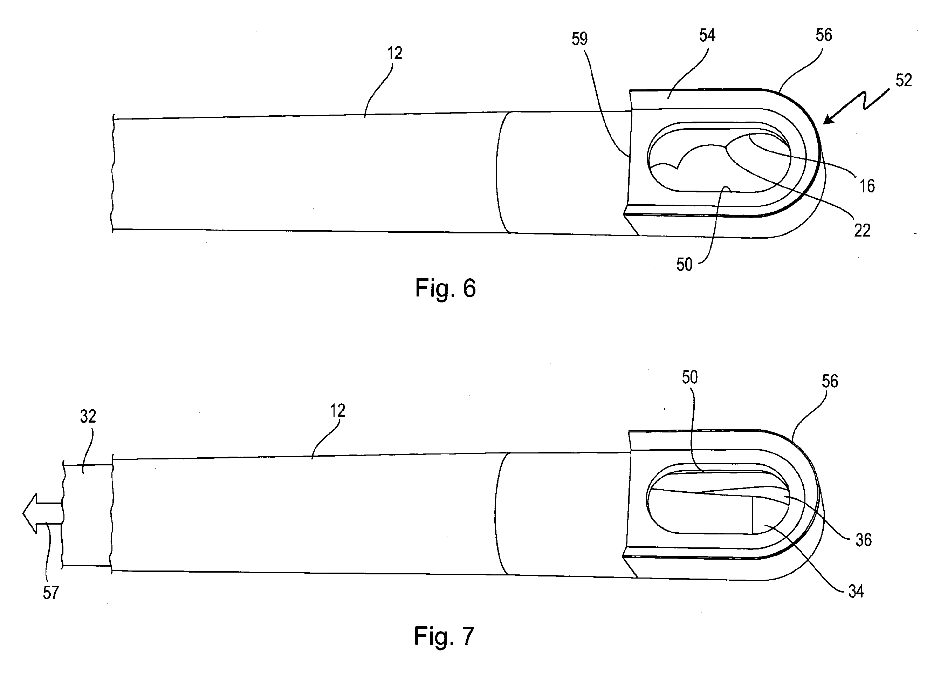

Aus der perspektivischen Darstellung von Fig. 6 ist ersichtlich, dass über das weitere Fenster 50 ein Zugang zum Innenraum des Außenschaftes 12 ermöglicht ist.

Aus Fig. 7 ist ersichtlich, dass, wenn der Innenschaft 32 eingeschoben ist, die Öffnungen 36, 37 und 38 auch im Bereich des weiteren Fensters 50 liegen.

Werden nun von der Trennkante 56 Knorpelstücke, auch relativ große Knorpelstücke abgetrennt, werden diese aufgrund des Unterdruckes durch die Unterdruckquelle 44 durch das weitere Fenster 50 in Richtung Innenraum des Außenschaftes 12 bewegt und dann durch die rotierenden Schneiden 40 der Öffnungen 36, 37 und 38 im Innenschaft in kleinste Stücke zerteilt. Wenn diese Stücke dann ausreichend klein sind, können diese über die Öffnungen 36, 37 und 38 in den Innenschaft 32 eingesaugt und abgeführt werden.

Ist die Kürette 52 mit der Trennkante 56 auf einen Knorpelbereich aufgesetzt und bis auf die Tiefe des Knochens eingedrückt, sind diese dadurch abgetrennten Knorpelbereiche in dem Raum "unter" der Kürette 52 zunächst gefangen und können nicht in das Gelenk entweichen. Sind diese Knorpelstücke dann in ausreichend kleine Stücke zerteilt, können sie über den Innenschaft 32 abgesaugt werden.

Die Trennkante 56 kann als geschlossene oval um das ovale Fenster 50 umlaufende Kante ausgebildet sein.

Im dargestellten Ausführungsbeispiel ist diese Trennkante 56 nach proximal offen.

Dies erleichtert der Handhabungsperson das Ansetzen der Kürette 52 des Instrumentes 10 an einer Defektstelle.

So kann der Operateur bspw. zunächst den distal geschlossenen Bereich der Trennkante 56 an die Defektstelle heranschieben und so ansetzen, dass diese etwas hinter dem vom Operateur aus gesehen, entferntesten Bereich der Defektstelle zum Liegen kommt und diese dabei diesem Bereich umrundet. Dann kann er nach und nach die Trennkante 56 in das Knorpelgewebe eindrücken. Dies kann er zumindest teilweise über das proximalseitig offene Ende 59 des Profiles einsehen.

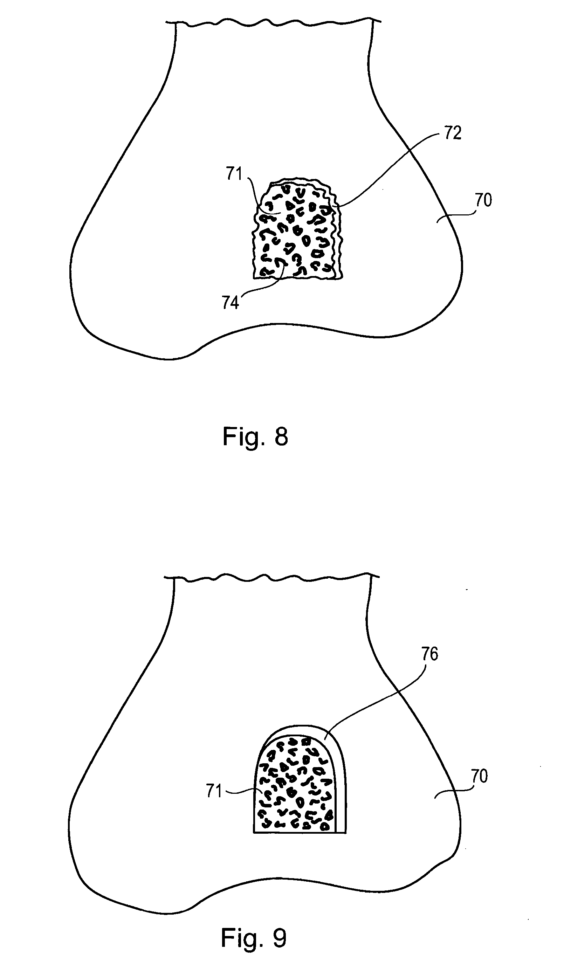

In Fig. 8 ist stark schematisch ein Oberschenkelknochen 70 im Bereich eines Kniegelenkes dargestellt.

Dort war ein Defekt 71 vorhanden, der zunächst mit dem "Shaver-Blade" des Instrumentes 10 bearbeitet worden ist. Im Bereich des Defektes 71 entsteht eine relativ unscharfe umfängliche Kante 72 an dem umrundenden Gewebe des Knochens 70. Das Knochenmaterial 74 ist bereits freigelegt.

Fig. 9 zeigt nun den Defekt 71 nach dem die Kürette 52 angesetzt, eingedrückt und das randseitige Knorpelgewebe bis herunter auf das Knochenmaterial 74 entfernt worden ist.

Dabei entstand durch die glatte und scharfe Trennkante 56 der Kürette 52 eine deren Geometrie entsprechende scharfe etwa im rechten Winkel von der Knochenfläche hochstehende Kante 76.

Das ist Ziel einer gelungenen Präparation einer Defektstelle an die ein entsprechend zugeschnittenes Transplantat eingesetzt werden soll. Diese glatte, etwa rechtwinklig hochstehende Kante 76 ist ein entscheidender Faktor für ein alsbaldiges festes und übergangsfreies Anwachsen eines Implantates. Nach einer gewissen Zeit ist dann der Defekt 71 vollständig und narbenfrei zugewachsen. Dann können wieder die erheblichen Belastungen, die insbesondere bei einem Kniegelenk in diesem Bereich einwirken, wieder ertragen werden.

Wie bereits erwähnt, kann die Kontur 54 der Trennkante 56 der Kürette auch geschlossen, oval oder rund sein. Die Fläche der Trennkante kann auch entsprechend gekrümmt verlaufen, wenn an extrem gekrümmten Knochenstellen solche Defekte auftreten, so dass dann die Kontur der Kürette bzw. deren Trennkante 56 schon dieser Knochenkontur angepasst sein kann.

## Patentansprüche

1. Medizinisches Instrument zum Abtrennen von Gewebe und Knorpel mit einem Außenschaft (12), der im Bereich seines distalen Endes (14) zumindest ein erstes Fenster (16) mit zumindest einer Schneide (20) aufweist,
mit einem im Außenschaft (12) aufgenommenen um seine Längsachse (33) rotierbaren Innenschaft (32), der an seinem distalen Ende (34), im Bereich des ersten Fensters (16) des Außenschafts (12) zumindest eine Öffnung (36, 37, 38) mit einer Schneide (40) aufweist, die mit der zumindest einen Schneide (20) des Außenschafts (12) schneidend zusammenwirkt, wobei der hohle Innenschaft (32) an eine Unterdruckquelle (44) anschließbar ist, wobei
am Außenschaft (12) im Bereich des distalen Endes (14) ein weiteres Fenster (50) ausgespart ist, **dadurch gekennzeichnet, dass** das weitere Fenster (50) am distalen Ende des Aussenschafts von einer Kürette (52) umgeben ist.

2. Medizinisches Instrument nach Anspruch 1, **dadurch gekennzeichnet, dass** die Kürette (52) von einer Außenfläche (58) des Außenschafts (12) radial vorsteht.

3. Medizinisches Instrument nach Anspruch 1 oder 2, **dadurch gekennzeichnet, dass** die Kontur (54) der Kürette (52), radial von außen auf den Außenschaft (12) gesehen, U-förmig ist.

4. Medizinisches Instrument nach Anspruch 3, **dadurch gekennzeichnet, dass** das offene Ende (59) der U-Kontur proximalseitig liegt.

5. Medizinisches Instrument nach einem der Ansprüche 1 bis 4, **dadurch gekennzeichnet, dass** eine Trennkante (56) der Kürette (52) in einer Ebene liegt.

6. Medizinisches Instrument nach Anspruch 5, **dadurch gekennzeichnet, dass** die Trennkante (56) der Kürette (52), um eine solche Höhe radial von der Außenfläche (58) des Außenschafts (12) vorsteht, die zumindest der Dicke einer abzutrennenden Knorpelschicht entspricht.

7. Medizinisches Instrument nach einem der Ansprüche 1 bis 6, **dadurch gekennzeichnet, dass** der Außenschaft (12) ein einziges erstes Fenster (16) aufweist, das diametral gegenüberliegend zur Kürette (52) angeordnet ist.

8. Medizinisches Instrument nach einem der Ansprüche 1 bis 7, **dadurch gekennzeichnet, dass** das erste Fenster (16) im Außenschaft (12) durch einen von proximal nach distal radial nach innen geneigten Schrägschnitt (24) des hohlen Außenschaftes (12) ausgebildet ist.

9. Medizinisches Instrument nach Anspruch 8, **dadurch gekennzeichnet, dass** der Innenschaft (32) im Bereich des Schrägschnittes (24) aus dem ersten Fenster (16) ragt.

10. Medizinisches Instrument nach einem der Ansprüche 1 bis 9, **dadurch gekennzeichnet, dass** eine Umfangskante (18) des ersten Fensters (16) im Außenschaft (12) von dieser Umfangskante (18) hochstehende Zacken (22) aufweist.

11. Medizinisches Instrument nach einem der Ansprüche 1 bis 10, **dadurch gekennzeichnet, dass** der rotierbare Innenschaft (32) mehrere Öffnungen (36, 37, 38) aufweist.

12. Medizinisches Instrument nach Anspruch 11, **dadurch gekennzeichnet, dass** der Innenschaft (32) drei Öffnungen (36, 37, 38) aufweist.

13. Medizinisches Instrument nach einem der Ansprüche 1 bis 12, **dadurch gekennzeichnet, dass** der Außenschaft (12) am proximalen Ende ein Kopplungsstück (26) aufweist, durch das der Innenschaft (32) von proximal führbar ist.

14. Medizinisches Instrument nach einem der Ansprüche 1 bis 13, **dadurch gekennzeichnet, dass** der Innenschaft (32) am proximalen Ende ein Anschlussstück (42) aufweist, über das er mit einer Unterdruckquelle (44) verbindbar ist.

15. Medizinisches Instrument nach Anspruch 14, **dadurch gekennzeichnet, dass** das Anschlussstück (42) über Kupplungselemente (46) mit einem Antrieb kuppelbar ist, der den Innenschaft (32) um dessen Längsachse (33) relativ zum stationären Au-βenschaft (12) dreht.

## Claims

1. Medical instrument for separating tissue and cartilage, comprising an outer shaft (12) which, in the area of it distal end (14) has at least a first window (16) with at least one cutting edge (20), an inner shaft (32) which is received in the outer shaft (12) is rotatable about its longitudinal axis (33) and has, at it distal end (34), in the area of the first window (16) of the outer shaft (12) at least one opening (36, 37, 38) with a cutting edge (40), that cooperates in a cutting action with the at least one cutting edge (20) of the outer shaft (12), wherein the hollow inner shaft (32) can be attached to a vacuum source (44) and wherein, in the outer shaft (12), in the area of the distal end (14) a further window (50) is cut out, **characterized in that** the further window (50) at the distal end of the outer shaft is surrounded by a curette (52).

2. Medical instrument of claim 1, **characterized in that** the curette (52) protrudes radially from an outer face (58) of the outer shaft (12).

3. Medical instrument of claims 1 or 2, **characterized in that** the contour (54) of the curette (52) is U-shaped when the outer shaft (12) is viewed radially from the outside.

4. Medical instrument of claim 3, **characterized in that** the open end (59) of the U-shaped contour lies at the proximal side.

5. Medical instrument of anyone of claims 1 through 4, **characterized in that** a separating edge (56) of the curette (52) lies in a single plane.

6. Medical instrument of claim 5, **characterized in that** the separating edge (56) of the curette (52) protrudes radially from the outer face (58) of the shaft (12) by a height that corresponds at least to the thickness of a layer of cartilage that is to be removed.

7. Medical instrument of anyone of claims 1 through 6, **characterized in that** the outer shaft (12) has a single first window (16), which is arranged laying diametrically opposite the curette (52)

8. Medical instrument of anyone of claims 1 through 7, **characterized in that** the first window (16) in the outer shaft (12) is formed by an oblique cut (24) of the hollow outer shaft (12), that is inclined radially inwards in the proximal to distal direction.

9. Medical instrument of claim 8, **characterized in that** the inner shaft (32) protrudes from the first window (16) in the area of the oblique cut (24).

10. Medical instrument of anyone of claims 1 through 9, **characterized in that** a circumferential rim (18) of the first window (16) in the outer shaft has teeth (22) standing vertically from this circumferential rim (18).

11. Medical instrument of anyone of claims 1 through 10, **characterized in that** the rotatable inner shaft (32) has a plurality of openings (36, 37, 38).

12. Medical instrument of claim 11, **characterized in that** the inner shaft (32) has three openings (36, 37, 38).

13. Medical instrument of anyone of claims 1 through 12, **characterized in that** the outer shaft (12) has, at the proximal end, a coupling piece (26) through which the inner shaft (32) can be guided from the proximal direction.

14. Medical instrument of anyone of claims 1 through 13, **characterized in that** the inner shaft (32) has at the proximal end, an attachment piece (42) via which it can be connected to a vacuum source (44).

15. Medical instrument of claim 14, **characterized in that** the attachment piece (42) can be coupled via coupling elements (46) to a drive which rotates the inner shaft (32) about its longitudinal axis (33) relative to the stationary outer shaft (12).

## Revendications

1. Instrument médical pour la séparation de tissu et de cartilage avec un arbre externe (12) qui présente dans la région de son extrémité distale (14) au moins une première fenêtre (16) avec au moins une lame (20), avec un arbre interne (32) reçu dans l'arbre externe (12) pouvant tourner autour de son axe longitudinal (33) qui présente à son extrémité distale (34), dans la région de la première fenêtre (16) de l'arbre externe (12), au moins une ouverture (36, 37, 38) avec une lame (40) qui coopère de manière coupante avec l'au moins une lame (20) de l'arbre externe (12), dans lequel l'arbre interne creux (32) peut être raccordé à une source de dépression (44), dans lequel
une autre fenêtre (50) est évidée sur l'arbre externe (12) dans la région de l'extrémité distale (14), **caractérisé en ce que** l'autre fenêtre (50) est entourée par une curette (52) à l'extrémité distale de l'arbre externe.

2. Instrument médical selon la revendication 1, **caractérisé en ce que** la curette (52) fait saillie radialement d'une surface externe (58) de l'arbre externe (12).

3. Instrument médical selon la revendication 1 ou 2, **caractérisé en ce que** le contour (54) de la curette (52) est en forme de U, vu radialement de l'extérieur sur l'arbre externe (12).

4. Instrument médical selon la revendication 3, **caractérisé en ce que** l'extrémité ouverte (59) du contour en U se situe du côté proximal.

5. Instrument médical selon une des revendications 1 à 4, **caractérisé en ce qu'**une arête de séparation (56) de la curette (52) se situe dans un plan.

6. Instrument médical selon la revendication 5, **caractérisé en ce que** l'arête de séparation (56) de la curette (52) fait saillie d'une telle hauteur radialement de la surface externe (58) de l'arbre externe (12) qui correspond au moins à l'épaisseur d'une couche de cartilage à séparer.

7. Instrument médical selon une des revendications 1 à 6, **caractérisé en ce que** l'arbre externe (12) présente une seule première fenêtre (16) qui est disposée de manière diamétralement opposée à la curette (52).

8. Instrument médical selon une des revendications 1 à 7, **caractérisé en ce que** la première fenêtre (16) est réalisée dans l'arbre externe (12) par une coupe en biais (24) de l'arbre externe creux (12) inclinée radialement vers l'intérieur de la direction proximale vers la direction distale.

9. Instrument médical selon la revendication 8, **caractérisé en ce que** l'arbre interne (32) dépasse de la première fenêtre (16) dans la région de la coupe en biais (24).

10. Instrument médical selon une des revendications 1 à 9, **caractérisé en ce qu'**une arête périphérique (18) de la première fenêtre (16) présente des dents (22) élevées de cette arête périphérique (18) dans l'arbre externe (12).

11. Instrument médical selon une des revendications 1 à 10, **caractérisé en ce que** l'arbre interne rotatif (32) présente plusieurs ouvertures (36, 37, 38).

12. Instrument médical selon la revendication 11, **caractérisé en ce que** l'arbre interne (32) présente trois ouvertures (36, 37, 38).

13. Instrument médical selon une des revendications 1 à 12, **caractérisé en ce que** l'arbre externe (12) présente à l'extrémité proximale une pièce d'accouplement (26) à travers laquelle l'arbre interne (32) peut être guidé de la direction proximale.

14. Instrument médical selon une des revendications 1 à 13, **caractérisé en ce que** l'arbre interne (32) présente à l'extrémité proximale une pièce de raccordement (42) par le biais de laquelle il peut être relié à une source de dépression (44).

15. Instrument médical selon la revendication 14, **caractérisé en ce que** la pièce de raccordement (42) peut être accouplée à un entraînement qui fait tourner l'arbre interne (32) autour de son axe longitudinal (33) par rapport à l'arbre externe stationnaire (12) par le biais d'éléments d'accouplement (46).
